# EUROPÄISCHE PATENTSCHRIFT

(11) **EP 1 675 634 B1**
(45) Veröffentlichungstag und Bekanntmachung des Hinweises auf die Patenterteilung: **25.07.2007**
(21) Anmeldenummer: 04765983.4
(22) Anmeldetag: 18.10.2004
(51) Int. Cl.: A61M 5/32, A61B 5/00

(54) **KANÜLENHALTER**
CANNULA HOLDER
SUPPORT DE CANULE

(30) Priorität: 20.10.2003 DE 10348603; 18.11.2003 DE 20317798 U; 03.12.2003 DE 20318879 U
(43) Veröffentlichungstag der Anmeldung: 05.07.2006
(73) Patentinhaber: Klinika Medical GmbH, 61250 Usingen (DE)
(72) Erfinder: KITTA, Johann, Karl, 51545 Waldbröl-Herfen (DE); BRUCHMANN, Helmut, 51674 Wiehl (DE)
(74) Vertreter: KEIL & SCHAAFHAUSEN Patentanwälte
(86) Internationale Anmeldenummer: PCT/EP2004/011708
(87) Internationale Veröffentlichungsnummer: WO 2005/039677

(56) Entgegenhaltungen:
- EP-A- 0 290 176
- EP-A- 1 350 528
- US-A- 5 769 826
- US-A- 5 810 775
- US-A1- 2002 133 091
- US-B1- 6 171 262

## Beschreibung

Die Erfindung betrifft einen Kanülenhalter mit Kanülenentsorgung mit einem langgestreckten, an einer ersten Stirnseite eine verschließbare Öffnung aufweisenden Kanülenbehälter, mit einer an der gegenüberliegenden zweiten Stirnseite angeordneten Kanülenhalterung zur Aufnahme einer Kanüle, welche durch die Kanülenhalterung bis in den Kanülenbehälter hindurchragt, und mit einen Entriegelungsmechanismus zum Lösen der Kanüle nach dem Gebrauch. Anstelle der Kanüle kann auch ein Adapter in die Kanülenhalterung aufgenommen werden, welcher durch die Kanülenhalterung bis in den Kanülenbehälter hindurchragt und nach dem Gebrauch mit dem Entriegelungsmechanismus gelöst werden kann. Der Adapter kann bspw. auf eine bereits in der Vene des Patienten befindliche Kanüle aufgesteckt werden.

Derartige Kanülenhalter werden bei der medizinischen Versorgung von Patienten im Besonderen bei der venösen Blutentnahme benutzt. Sie sind zusammen mit einer Kanüle und einem Blutabnahmeröhrchen Teil eines meist dreistückigen Entnahmebestecks. Bei einer Blutabnahme wird in der Regel zunächst eine frische Kanüle an dem Kanülenhalter befestigt. Dazu verfügen die meisten handelsüblichen Kanülen über einen an der Kanüle angebrachten Adapter mit einem Außengewinde, welches in den Kanülenhalter eingedreht wird. Danach wird das aus dem Halter hervorstehende Ende der Kanüle in eine Vene des Patienten eingeführt. Auf das sich im Halter befindende Ende der Kanüle wird zum Auffangen des Blutes ein Blutabnahmeröhrchen aufgesetzt. Dieses kann je nach der erforderlichen Blutmenge oder den durchzuführenden Untersuchungen durch neue Röhrchen ersetzt werden. Nach erfolgter Blutabnahme wird die Kanüle aus der Vene entfernt und entsorgt. Bei der Entsorgung besteht allerdings die Gefahr, dass sich das Personal mit der Kanüle versehentlich eine Nadelstichverletzung zuzieht. Solch eine Verletzung kann besonders schwerwiegende Folgen haben, da das in der Kanüle enthaltene Blut des Patienten Träger von ansteckenden Krankheiten sein kann.

Um das Risiko solcher Verletzungen zu verringern, wird bspw. in der EP 0 315 306 A1 bei einer Spritze vorgeschlagen, die auf die Spritze aufgesteckte Kanüle nach dem Gebrauch in einen Behälter einzustecken und diesen zu verschließen. Da der Behälter beim Einstecken jedoch festgehalten werden muss, besteht gerade dabei eine besonders große Verletzungsgefahr.

Dieses Problem wird bei einer in der WO 93/18808 beschrieben Spritze vermieden, bei der die Kanüle nach dem Gebrauch in eine Frontkammer eingezogen wird, welche durch ein Diaphragma von der eigentlichen Spritzenkammer mit dem Kolben getrennt ist. Um einen Wiedergebrauch zu verhindern, kann nach dem Gebrauch durch tiefes Eindrücken des Kolbens ein automatisches Rückholsystem ausgelöst werden, durch das die Kanüle in die Frontkammer eingezogen wird. Dies kann allerdings nicht unmittelbar nach einer Blutabnahme erfolgen, da das Blut erst aus der Spritzenkammer entfernt werden muss, bevor der Kolben wieder tief eingedrückt werden kann. Dies ist typischerweise erst im Labor der Fall, so dass bis dahin noch ein erhebliches Verletzungsrisiko besteht. Außerdem kann es zu schmerzhaften Verletzungen beim Patienten kommen, wenn der Kolben während der Blutabnahme versehentlich zu weit eindrückt wird. Auch ist eine Mehrfachverwendung der Kanüle durch Aufstecken mehrerer Blutabnahmeröhrchen nicht möglich.

Die EP 0 943 352 A2 beschreibt einen Kanülenhalter, bei dem die Kanüle nach der Benutzung über einen Auslösemechanismus von dem Halter abgeworfen werden kann. Dazu positioniert der Benutzer den Kanülenhalter mit der kontaminierten Kanüle über einem entsprechenden Entsorgungsbehälter und betätigt einen Schieber, wodurch die Kanüle vom Halter gelöst wird und in den Behälter fällt. Es besteht jedoch der Nachteil, dass die Kanüle versehentlich auch neben den Behälter fallen kann und dann von Hand entsorgt werden muss. Dies kann leicht zu Nadelstichverletzungen führen. Gleiches gilt, wenn der Abwurfmechanismus zu einem unerwünschten Zeitpunkt ausgelöst wird. Schließlich kann es beim Abwerfen der Kanüle auch vorkommen, dass Blut von der Kanüle auf den - an sich wiederverwendbaren - Halter gelangt. Bei Wiederwendung des Halters besteht dann eine zusätzliche von diesem ausgehende Infektionsgefahr. Um dies zu verhindern, ist eine aufwendige Desinfizierung des Halters notwendig.

In der US 6,171,262 B1 wird ein Kanülenhalter mit einer Kanülenhalterung beschrieben, in der eine Kanüle fest eingesteckt ist. Die Kanülenhalterung wird durch einen an dem Kanülenhalter festgelegten Ring in einer Arbeitsposition gehalten, während die Kanüle zum Blutabnehmen in die Vene eines Patienten eingesteckt wird. Nach der Blutabnahme wird die Kanüle durch Drehen des Rings freigegeben und die Kanülenhalterung mit der Kanüle durch Federkraft in den Kanülenhalter eingezogen.

Aus der US 2002/0133091 A1 ist ein Kanülenhalter mit einem Adapter bekannt, auf das von außen eine Kanüle aufgesteckt werden kann. Der Adapter ist einteilig mit einer Kanülenhalterung verbunden, welche einen aus dem Kanülenhalter herausragen Schieber aufweist, mit dem die Kanülenhalterung in den Kanülenhalter zurückgezogen werden kann. In einer zweiten Ausführungsform ist ein Kanülenhalter mit einer Kanüle dargestellt, die einteilig mit einer Kanülenhalterung ausgebildet ist. Die Kanülenhalterung bildet einen Teil des Kanülenbehälters und kann mittels eines Schiebers nach der Blutentnahme nach außen abgestoßen werden.

Aus den US 5,810,775, US 5,769,826 und EP 0 290 176 A1 sind Kanülenhalter bekannt, an denen eine Kanülenhalterung mit Kanüle zur Blutentnahme festgelegt ist. Nach der Blutentnahme kann die Kanülenhalterung mit Kanüle durch jeweils unterschiedliche Arten von Schiebern von dem Kanülenhalter gelöst werden, so dass die Kanülenhalterung mit der Kanülen in den Kanülenhalter fällt oder durch Federkraft eingezogen wird.
Es ist Aufgabe der vorliegenden Erfindung, einen Kanülenhalter vorzuschlagen, bei welchem die Entsorgung der Kanüle nach der Benutzung besonders sicher, schnell und einfach erfolgt.

Diese Aufgabe wird bei einem Kanülenhalter der eingangs genannten Art erfindungsgemäß mit den Merkmalen des Anspruchs 1 gelöst. Dabei ist der Entriegelungsmechanismus, durch den die Kanüle und/oder der Adapter nach Betätigung des Entriegelungsmechanismus selbst in den Kanülenbehälter fällt, derart ausgebildet, dass er eine Verriegelungseinrichtung zum Verschließen des Kanülenbehälters an seiner zweiten Stirnseite aufweist. Vor dem Auslösen des Entriegelungsmechanismus wird die an der ersten Stirnseite des Kanülenhalters ausgebildete Öffnung, durch die die Blutabnahmeröhrchen auf die eine Seite der Kanüle oder des Adapters aufgesteckt werden, bspw. durch einen Stopfen, einen Schraubverschluss, eine Kappe oder dgl. Verschlussmittel verschlossen. Dies ist mit keiner Verletzungsgefahr verbunden, da dies auf der der Kanüle abgewandten Seite des Kanülenhalters erfolgt. Nach dem Auslösen des Entriegelungsmechanismus fällt die Kanüle bzw. der Adapter dann in den Kanülenbehälter, der so lang ausgebildet ist, dass er die Kanüle bzw. den Adapter vollständig aufnimmt. Dadurch werden jeder Kontakt mit der durch Blut kontaminierten Kanüle oder dem entsprechenden Adapter und insbesondere gefährliche Stichverletzungen vermieden. Vor dem Entsorgen ist durch die Verriegelungseinrichtung auch die zweite offene Seite des Kanülenhalters, an der die Kanüle bzw. der Adapter angeordnet war, verschlossen. Im Folgenden wird nur noch auf die Kanüle Bezug genommen, wobei die Kanüle jeweils auch durch einen Adapter bspw. zum Aufstecken einer Kanüle ersetzt sein kann.

Um ein unbeabsichtigtes Abfallen der Kanüle von dem Kanülenhalter zu verhindern, ist erfindungsgemäß eine Arretierung vorzusehen, welche die Kanüle an dem Kanülenbehälter festhält. Diese Arretierung ist dabei so ausgebildet, dass sie nur eine Bewegung der Kanüle in den Kanülenbehälter zulässt. Sie kann in den Entriegelungsmechanismus integriert sein.

Dazu kann die Kanülenhalterung mit einer Verankerung lösbar an dem Kanülenhalter fixiert sein. Insbesondere ist es vorteilhaft, wenn die Kanülenhalterung von der Innenseite des Kanülenbehälters an diesem derart festgelegt ist, dass die Kanüle von außen durch eine Öffnung in dem Kanülenhalter bspw. eingeschraubt werden kann. Nach einem Lösen der Verankerung fällt die Kanülenhalterung mit der eingeschraubten Kanüle dann von selbst in den Kanülenbehälter. Ein Abfallen der Kanüle wird so zuverlässig verhindert, weil die Kanülenhalterung von innen an dem Kanülenbehälter festgelegt ist und aus diesem nicht herausfallen kann.

Gemäß einer besonders bevorzugten Ausführungsform ist die Kanülenhalterung an dem Kanülenhalter einrastbar. Durch das Einrasten wird diese zum einen sicher fixiert. Zum anderen kann durch der Einrastung entgegenwirkende Kräfte diese Verankerung wieder leicht gelöst werden.

Erfindungsgemäß kann der Kanülenhalter an seiner zweiten Stirnseite eine Durchgangsöffnung für die Kanüle und mindestens eine Befestigungsöffnung für die Kanülenhalterung aufweisen, wobei die Kanülenhalterung in der Befestigungsöffnung insbesondere einrastbar ist. Dabei sind die Durchgangsöffnung für die Kanüle und die Befestigungsöffnungen bevorzugt separate Öffnungen. So kann bspw. die Kanülenhalterung mit in die bspw. an den Rändern verstärkte Durchgangsöffnung eingreifen, wodurch die Kanülenhalterung mit Kanüle besonders gut geführt und fest ausgerichtet wird. Es ist aber auch möglich, dass die Durchgangsöffnung und die Befestigungsöffnung eine gemeinsame Öffnung bilden. In einer bevorzugten Ausführung sind eine insbesondere zentrale Durchgangsöffnung und zwei sich auf verschiedenen Seiten der Durchgangsöffnung gegenüberliegende Befestigungsöffnungen vorgesehen. Dies ermöglicht sowohl eine stabile Befestigung als auch ein einfaches Lösen der Kanülenhalterung.

Als Befestigungsmittel kann die Kanülenhalterung mindestens einen flexiblen Arm mit einem vorzugsweise keilförmigen Vorsprung zum Einrasten an der Kanülenhalterung aufweisen. Dabei sind der Arm und der Vorsprung so angeordnet, dass der Arm durch die Befestigungsöffnung in dem Kanülenhalter von innen durchgreift und der Vorsprung sich von außen an dem Kanülenhalter einhakt. Durch Ausüben von Druck auf den Vorsprung hebt sich dieser von dem Rand der Befestigungsöffnung ab und die Verankerung wird gelöst. Der Vorsprung kann allerdings auch nur der Führung dienen.

Dazu weist der Entriegelungsmechanismus gemäß der vorliegenden Erfindung vorzugsweise mindestens einen Schieber auf, welcher durch Verschieben die Verankerung der Kanülenhalterung an dem Kanülenhalter löst. Durch einen solchen Schieber kann die zum Lösen der Verankerung aufzuwendende Kraft einfach und zielgerichtet aufgebracht werden.

Dies ist besonders dann gut möglich, wenn der Schieber eine Führung aufweist, welche an der Verankerung der Kanülenhalterung angreift. Dies können bspw. ein schräg zulaufender Arm, eine in dem Schieber entsprechend ausgebildete Nut oder dgl. sein, wobei ein Schieber erfindungsgemäß auch mehrere an verschiedenen Verankerungspunkten angreifende Führungen aufweisen kann, bspw. zwei Arme mit entsprechend zulaufenden Nuten. In einem solchen Fall ist die Montage des Entriegelungsmechanismus besonders einfach, weil dieser nur wenige Einzelteile aufweist.

Alternativ können erfindungsgemäß auch zwei Schieber vorgesehen sein, welche vorzugsweise je an einem flexiblen Arm angreifen.

Damit der Schieber einfach von Hand durch Drücken von der Seite bedienbar ist, kann er auch über den Rand des Kanülebehälters vorstehen. Dann ist er besonders einfach bspw. mit dem Daumen zu betätigen, während der Halter von der Hand umfasst wird. Durch diese Ein-Hand-Bedienung kann das Verletzungsrisiko noch einmal deutlich reduziert werden.

Statt eines nach außen vorstehenden Schiebers kann der Entriegelungsmechanismus erfindungsgemäß auch eine auf dem Kanülenbehälter drehbar angeordnete Kappe aufweisen, in welcher der mindestens eine Schieber derart aufgenommen ist, dass der Schieber durch Verdrehen der Kappe bewegt wird. Dabei ist es möglich, Kappe und Schieber einteilig auszubilden. Dann weist die Kappe selbst eine Führung derart auf, dass die Verankerung der Kanülenhalterung bei der Drehbewegung gelöst wird. Die Drehung kann dabei insbesondere nach rechts oder links erfolgen, was für eine einfache und unkomplizierte Anwendung vorteilhaft ist.

Alternativ kann die Kappe gemäß der vorliegenden Erfindung eine Führung aufweisen, in welche ein Führungselement des Schiebers eingreift. Für zwei Schieber kann dies bspw. eine doppelringförmige Nut sind.

Erfindungsgemäß weist der Entriegelungsmechanismus eine Verschlusseinrichtung zum Verschließen des Kanülenbehälters auf. Diese kann besonders einfach durch einen Schieber gebildet werden. Der oder die Schieber können besonders gut durch eine drehbare Kappe betätigt werden, wobei die Verschlusswirkung vorzugsweise sowohl durch Links- als auch durch Rechtsdrehung erreicht wird. Dadurch wird zuverlässig erreicht, dass der als Entsorgungsbehälter dienende Kanülenhalter immer verschlossen ist, ohne dass die zweite Stirnseite noch mit einem zusätzlichen Handgriff bspw. durch Aufbringen einer Kappe abgedichtet werden muss.

Gemäß einem weiteren Aspekt der vorliegenden Erfindung soll auch die Handhabung der Kanülenhalterung während der Blutabnahme verbessert werden. Dazu ist erfindungsgemäß ein Kanülenhalter mit einem an einer ersten Stirnseite eine insbesondere verschließbare Öffnung aufweisenden Kanülenbehälter, mit einer an der gegenüberliegenden zweiten Stirnseite angeordneten Kanülenhalterung zur Aufnahme einer Kanüle und/oder eines Adapters zum Aufstecken einer Kanüle vorgesehen, bei dem die Kanülenhalterung in eine radiale Richtung von der Mittelachse des Kanülenhalters versetzt angeordnet ist. Dies führt dazu, dass die Kanüle oder der Adapter nicht in der Mitte des Kanülenhalters nach oben vorsteht, sondern zum Rand des Kanülenhalters versetzt ist.

Da die Kanüle in der Regel in einem flachen Winkel bspw. an einem Arm des Patienten angesetzt werden muss, um in die Vene eingestochen werden zu können, wird bei einer in der Mitte des Kanülenhalters angeordneten Kanülenhalterung mit Kanüle der Einstichwinkel durch den am Arm des Patienten anliegenden Kanülenhalter begrenzt. Dies führt in der Praxis dazu, dass die Kanüle beim Einstechen in die Vene des Patienten unter Aufbringen von Kraft abgebogen werden muss, wodurch sich die Verletzungsgefahr und die Schwierigkeiten bei der Blutabnahme erhöhen. Wenn die Kanüle erfindungsgemäß zum Rand des Kanülenhalters hin versetzt angeordnet ist, sind bei entsprechender Haltung des Kanülenhalters kleinere Einstichwinkel möglich, so dass die Kanüle insgesamt flacher in den Arm eingestochen werden kann. Dadurch wird die Handhabung des Kanülenhalters bei der Blutabnahme erheblich verbessert. Diese erfindungsgemäße Lösung, die auch für sich den Gegenstand der vorliegenden Erfindung bildet, lässt sich besonders vorteilhaft mit der vorbeschriebenen Lösung zur Entsorgung der Kanüle verbinden, indem der Entriegelungsmechanismus derart mit der versetzt angeordneten Kanülenhalterung zusammenwirkt, dass die Kanüle und/oder der Adapter nach Betätigung des Entriegelungsmechanismus in den Kanülenbehälter fällt. Die vorbeschriebenen Ausgestaltungen können dabei entsprechend Anwendung finden.

Gemäß einer erfindungsgemäßen Ausführungsform kann die Kanüle oder der Adapter in der Kanülenhalterung drehbar aufgenommen sein, um den Kanülenschliff in jede gewünschte Position drehen zu können. Dies ist besonders bei einem Kanülenhalter mit versetzt angeordneter Kanüle vorteilhaft, weil der Kanülenhalter mit Kanüle dann nicht mehr rotationssymmetrisch aufgebaut ist und es eine Vorzugsrichtung für den Kanülenschliff gibt. Dies ist einfach mit einer Steckverbindung zu lösen. Alternativ kann durch eine entsprechende Führung an der Kanüle und der Kanülenhalterung auch die richtige Ausrichtung des Kanülenschliffs vorgegeben werden.

Weitere Merkmale, Vorteile und Anwendungsmöglichkeiten der Erfindung ergeben sich aus der nachfolgenden Beschreibung von Ausführungsbeispielen und der Zeichnung. Dabei gehören alle beschriebenen und/oder bildlich dargestellten Merkmale für sich oder in beliebiger Kombination zum Gegenstand der Erfindung, auch unabhängig von ihrer Zusammenfassung in den Ansprüchen oder deren Rückbezügen.

Es zeigen:
- Fig. 1: einen Querschnitt durch eine erste Ausführungsform eines erfindungsgemäßen Kanülenhalters mit eingesetzter Kanüle;
- Fig. 2: einen um 90° gedrehten Querschnitt durch den Kanülenhalter gemäß Fig. 1 mit einer in den Kanülenhalter aufgenommenen Kanüle;
- Fig. 3: eine Draufsicht auf den Kanülenhalter gemäß Fig. 1;
- Fig. 4: eine Draufsicht auf die zweite Stirnseite des Kanülenbehälters des Kanülenhalters gemäß Fig. 1;
- Fig. 5: einen Querschnitt durch eine erfindungsgemäße Kanülenhalterung;
- Fig. 6: eine Draufsicht auf die Kanülenhalterung gemäß Fig. 5;
- Fig. 7: einen Querschnitt durch eine zweite Ausführungsform eines erfindungsgemäßen Kanülenhalters mit eingesetzter Kanüle;
- Fig. 8: einen um 90° gedrehten Querschnitt durch den Kanülenhalter gemäß Fig. 7 mit einer in den Kanülenhalter aufgenommenen Kanüle;
- Fig. 9: eine Detailansicht des einen Endes des Kanülenhalters in der Ansicht gemäß Fig. 7;
- Fig. 10: eine Detailansicht des einen Endes des Kanülenhalters in der Ansicht gemäß Fig. 8;
- Fig. 11: eine Seitenansicht, eine Frontansicht und eine Draufsicht eines erfindungsgemäßen Schiebers;
- Fig. 12: eine erfindungsgemäße Kappe in der Ansicht von unten;
- Fig. 13: einen Querschnitt durch eine Kappe gemäß Fig. 7;
- Fig. 14: eine Draufsicht auf einen Kanülenhalter gemäß Fig. 7;
- Fig. 15: einen Querschnitt durch eine dritte Ausführungsform eines erfindungsgemäßen Kanülenhalters mit eingesetzter Kanüle;
- Fig. 16: einen um 90° gedrehten Querschnitt durch den Kanülenhalter gemäß Fig. 15 mit einer in den Kanülenhalter aufgenommenen Kanüle;
- Fig. 17: eine Detailansicht des einen Endes des Kanülenhalters in der Ansicht gemäß Fig. 15;
- Fig. 18: einen Querschnitt durch eine vierte Ausführungsform eines erfindungsgemäßen Kanülenhalters mit eingesetzter Kanüle;
- Fig. 19: einen um 90° gedrehten Querschnitt durch den Kanülenhalter gemäß Fig. 18 mit einer in den Kanülenhalter aufgenommenen Kanüle;
- Fig. 20: eine Detailansicht des einen Endes des Kanülenhalters in der Ansicht gemäß Fig. 18;
- Fig. 21: eine Draufsicht auf die zweite Stirnseite des Kanülenhalters gemäß Fig. 20;
- Fig. 22: eine Ansicht eines erfindungsgemäßen Schiebers des Kanülenhalters gemäß Fig. 18;
- Fig. 23: einen Querschnitt durch eine Kappe des Kanülenhalters gemäß Fig. 18;
- Fig. 24: eine Seitenansicht, eine um 90° gedrehte Seitenansicht und eine Draufsicht einer Kanülenhalterung des Kanülenhalters gemäß Fig. 18;
- Fig. 25: eine erfindungsgemäße Kanülenhalterung gemäß einer weiteren Ausführungsform der vorliegenden Erfindung;
- Fig. 26: eine erfindungsgemäße Kanüle für die Kanülenhalterung gemäß Fig. 25;

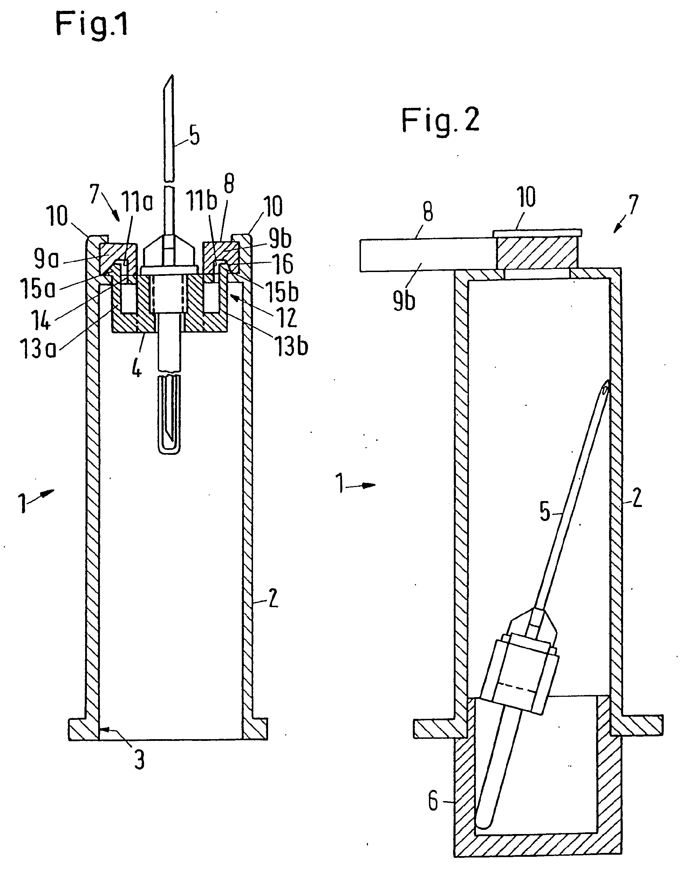

Der in Fig. 1 dargestellte Kanülenhalter 1 weist einen langgestreckten, zylinderförmigen Kanülenbehälter 2 auf, der auf einer ersten, in der Darstellung unteren Stirnseite eine verschließbare Öffnung 3 aufweist. An der gegenüberliegenden zweiten Stirnseite des Kanülenbehälters 2 ist eine von innen montierte Kanülenhalterung 4 angeordnet, in die eine handelsübliche Kanüle 5 eingeschraubt ist, welche durch die Kanülenhalterung 4 bis in den Kanülenbehälter 2 hindurchragt.

Zur Blutabnahme werden das freie Ende der Kanüle 5 in die Vene des Patienten eingestochen und auf das in dem Kanülenbehälter 2 befindliche Ende der Kanüle 5 durch die Öffnung 3 ein nicht dargestelltes Blutabnahmeröhrchen aufgesteckt. Nach der Blutabnahme wird das Blutabnahmeröhrchen wieder abgezogen und die Kanüle 5 aus der Vene des Patienten gezogen. Der Kanülenhalter 1 mit der Kanüle 5 muss nun entsorgt werden. Um eine Verletzung durch einen Nadelstich auszuschließen, ist daher zunächst die Kanüle 5 in den Kanülenbehälter 2 einzubringen.

Dazu wird die Öffnung 3 zunächst mit einer Kappe 6 verschlossen, die einfach aufgesteckt wird (vgl. Fig. 2). Danach wird ein Entriegelungsmechanismus 7 betätigt, welcher an der zweiten Stirnseite des Kanülenbehälters 2 angebracht und derart ausgebildet ist, dass die Kanüle 5 nach Betätigung des Entriegelungsmechanismus 7 in den Kanülenbehälter 2 fällt.

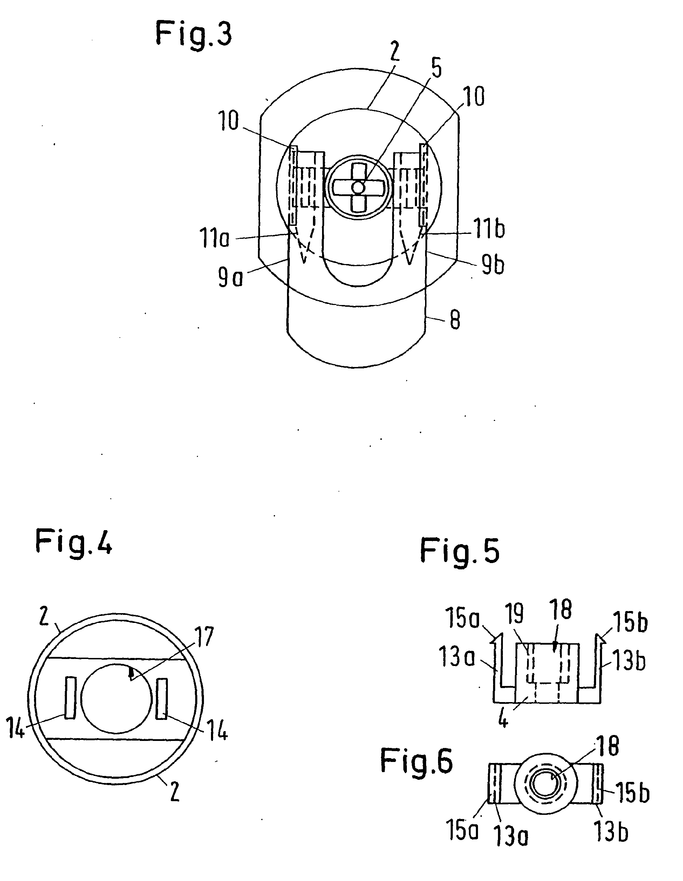

Der Entriegelungsmechanismus 7 weist einen Schieber 8 mit zwei Armen 9a, 9b auf (vgl. Fig. 3), welcher durch einen an dem Kanülenbehälter 2 ausgebildeten, abwinkelten Vorsprung 10 gehalten und geführt wird. Der Vorsprung 10 ist an zwei einander gegenüberliegenden Seiten des Kanülenbehälters 2 ausgebildet. In ihrer dem Kanülenbehälter 2 zugewandten Unterseite weisen die Arme 9a, 9b als Führungen dienende Nuten 11a, 11b auf.

Diese Nuten 11a, 11b greifen an einer Verankerung 12 der Kanülenhalterung 4 an, welche durch zwei flexible Arme 13a und 13b gebildet wird, wobei die Arme 13a, 13b an der Seite der Kanülenhalterung 4 ausgebildet und jeweils durch eine in der zweiten Stirnseite des Kanülenbehälters 2 ausgebildete Befestigungsöffnung 14 durchgeführt sind. An dem aus dem Kanülenbehälter 2 herausragenden Ende der Arme 13a, 13b sind jeweils nach außen gerichtete, keilförmige Vorsprünge 15a, 15b ausbildet, welche mit einem seitlich nach außen gerichteten Absatz an der Außenseite des Kanülenbehälters einrasten und zu ihrem Ende hin keilförmig zulaufen.

Die keilförmigen Vorsprünge 15a, 15b greifen in die Nuten 11a, 11b des Schiebers 8 ein. Dabei weisen die Nuten 11a, 11b jeweils eine der Keilform der Vorsprünge 15a, 15b entsprechend abgeschrägte Seite 16 auf, welche an den Vorsprüngen 15a, 15b anliegt. Die Nuten 11a, 11b beginnen an dem freien Ende der Arme 9a, 9b mit konstanter Breite und laufen sich verjüngend auf den die beiden Arme 9a, 9b verbindenden Abschnitt des Schiebers 8 zu, wie in Fig. 3 zu erkennen ist. Wenn der im Wesentlichen hufeisenförmige Schieber 8 von seinem über den Rand des Kanülenbehälters 2 herausstehenden Ende zur Mitte hin gedrückt wird, üben die abgeschrägten Seiten 16 der sich verjüngenden Nuten 11a, 11b eine Kraft auf die Vorsprünge 15a, 15b aus, so dass die flexiblen Arme 13a, 13b nach innen zusammengedrückt werden, bis die Absätze der Vorsprünge 15a, 15b ihren Halt am Rand der Befestigungsöffnungen 14 verlieren und die Kanülenhalterung 4 zusammen mit der Kanüle 5 in den Kanülenbehälter 2 fällt. Dazu sind die Nuten 11a, 11b und die Befestigungsöffnungen 14 breiter ausgebildet als die Vorsprünge 15a, 15b. Die Nuten 11a, 11b sowie die Breite der Kanülenhalterung 4 verhindern dabei, dass die Kanüle 5 mit der Kanülenhalterung 4 von dem Kanülenhalter 1 abfällt.

Wenn der Schieber 8 weitergedrückt wird, deckt sein die Arme 9a, 9b verbindender Abschnitt die Befestigungsöffnungen 14 sowie eine zwischen den Befestigungsöffnungen 14 ausgebildete, zentrale Durchgangsöffnung 17 für die Kanüle ab, die in der die zweite Stirnseite des Kanülenbehälters 2 darstellenden Fig. 4 zu erkennen sind. Dadurch wird der Kanülenhalter 1 zuverlässig verschlossen und ein Austreten der in den Kanülenbehälter 2 aufgenommenen Kanüle sicher ausgeschlossen.

Fig. 5 zeigt den Kanülenhalter 4 mit den flexiblen Armen im 13a, 13,b im Querschnitt. Der zentrale Abschnitt des Kanülenhalters 4 weist dabei einen Durchlass 18 mit im montierten Zustand von außen zugänglichem Innengewinde 19 auf, in welches handelübliche Kanülen 5 eingeschraubt werden können. Der zentrale Abschnitt der zylinderförmigen Kanülenhalterung 4 ist mit seinem Au- . ßendurchmesser dem Innendurchmesser der Durchgangsöffnung 17 angepasst, so dass die Kanülenhalterung 4 dort an dem Kanülenbehälter 2 anliegt und stabilisiert wird. Wie Fig. 6 zu entnehmen, sind die flexiblen Arme 13a, 13b über Stege mit dem Kanülenhalter verbunden.

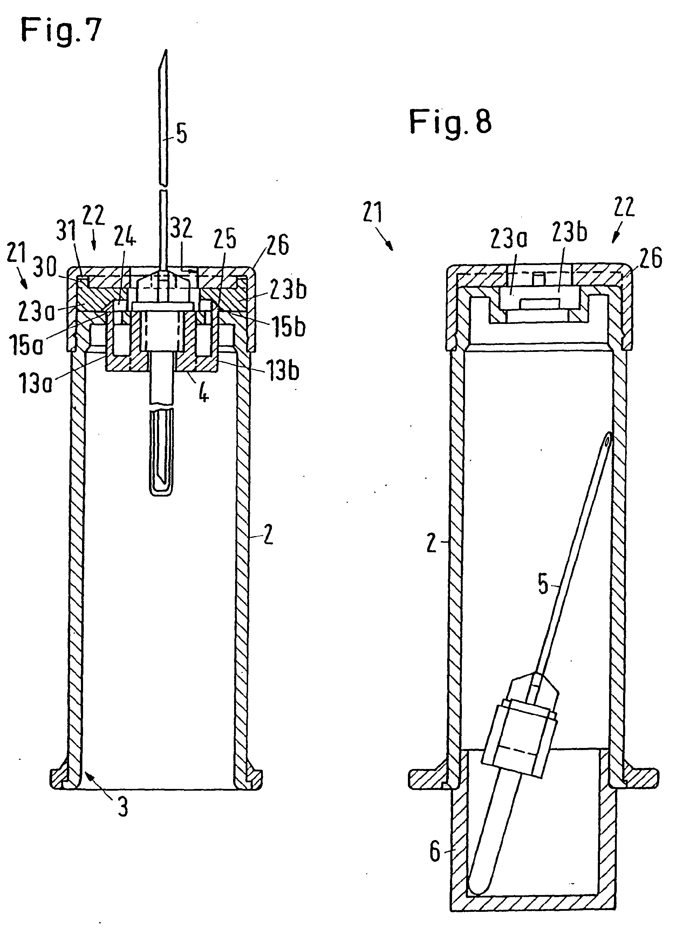

In Fig. 7 wird eine zweite Ausführungsform eines erfindungsgemäßen Kanülenhalters gezeigt. Der Kanülenhalter 21 ist im grundsätzlichen Aufbau dem Kanülenhalter 1 der oben beschriebenen ersten Ausführungsform ähnlich. Insbesondere die Kanülenhalterung 4 und der Kanülenbehälter 2 sind vergleichbar ausgebildet. Daher wird auf eine detaillierte Beschreibung dieser gleichen Teile verzichtet, die der Einfachheit halber mit denselben Bezugszeichen versehen sind.

Der Kanülenhalter 21 weist einen vom Kanülenhalter 1 verschiedenen Entriegelungsmechanismus 22 auf, der mit zwei Schiebern 23a und 23b ausgestattet ist, die baugleich ausgebildet sind. Wie Fig. 7 zu entnehmen ist, sind die Schieber 23a, 23b auf der zweiten Stirnseite des Kanülenbehälters 2 angeordnet, an der die Kanülenhalterung 4 von der Innenseite des Kanülenbehälters 2 wie bei der ersten Ausführungsform montiert ist. Dabei weist jeder Schieber 23a, 23b an seiner Unterseite eine Ausnehmung 24 auf, in welche je ein Vorsprung 15a, 15b der flexiblen Arme 13a, 13b der Kanülenhalterung 4 aufgenommen ist. Dabei liegt der keilförmige Vorsprung 15a, 15b an einer abgeschrägten Seite 25 der Ausnehmung 24 an.

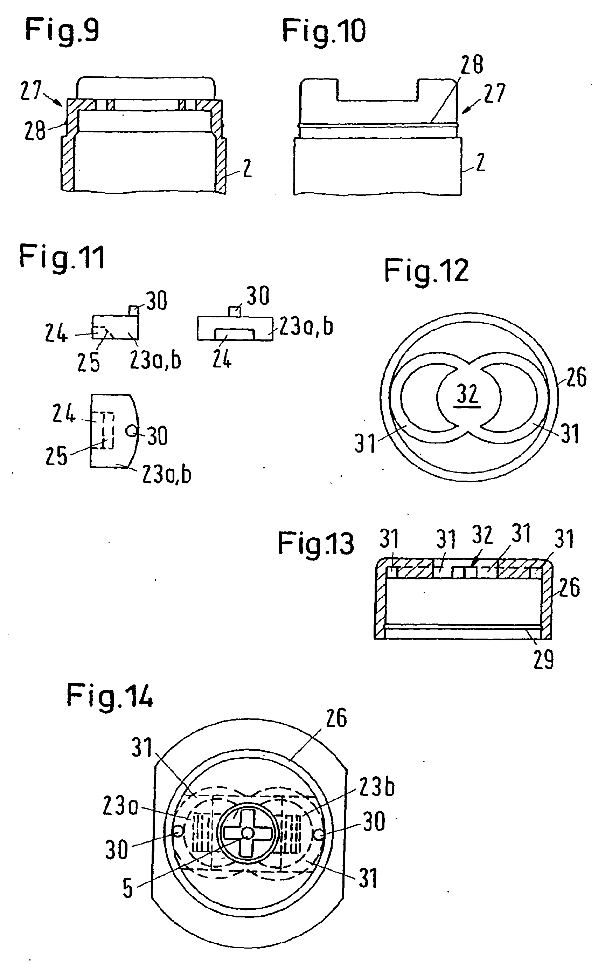

Der Schieber 23a, 23b ist im wesentlichen rechteckig ausgebildet, wobei eine Seite an die Rundung des Kanülenbehälters 2 angepasst ist. Damit kann der Schieber 23a, 23b vollständig auf der Stirnseite des Kanülenbehälters 2 aufliegen und steht insbesondere nicht über. Gehalten werden die beiden Schieber 23a, 23b durch eine Kappe 26 mit einem zentralen Durchgang 32 zum Durchführen der Kanüle. Die Kappe 26 ist von außen auf die Stirnseite des Kanülenhalters 21 aufgesetzt und umschließt und fixiert die Schieber 23a und 23b. Dazu ist der Kanülenbehälter 2 am oberen Seitenrand mit einem nach innen weisenden Absatz 27 versehen, auf den die Kappe 26 aufgesteckt werden kann. Um diese festzuhalten, ist im Bereich des Absatzes 27 auf dem Seitenrand des Gehäuses eine Erhebung 28 ausgebildet, welche in eine in der Kappe 26 entsprechend ausgebildete Nut 29 eingereift. Detailansichten des Absatzes 27 und der Kappe 26 zeigen die Fig. 9, 10 und 13.

In Betriebsstellung des Kanülenhalters 1 mit aufgeschraubter und einsatzbereiter Kanüle 5 ist der Schieber 23a, 23b derart zwischen der Kanüle 5 und dem Rand des Kanülenbehälters 2 angeordnet, dass der abgerundete Rand des Schiebers 23a, 23b mit dem Rand des Kanülenbehälters 2 deckungsgleich ist. Zum Betätigen des Schiebers 23a, 23b ist auf dessen Oberseite ein als Führungselement dienender Nippel 30 vorgesehen (vgl. Fig. 11), der in einer entsprechend ausgebildeten Nut 31 der Kappe 26 geführt ist. Die Nut 31 in der Kappe 26 ist, wie in Fig. 12 gezeigt, doppelringförmig ausgebildet, so dass der Nippel 30 jedes Schiebers 23a, 23b beim Verdrehen der Kappe 26 nach links oder rechts nach vorne bewegt wird und dabei den Schieber 23a, 23b mit nach vorne schiebt. Dabei drückt die abgeschrägte Seite 25 der Schieber 23a, 23b die Vorsprünge 15a, 15b der Kanülenhalterung 4 aus der Verankerung, so dass die Kanüle 5 mit der Kanülenhalterung in den Kanülenbehälter 2 fällt (vgl. Fig. 8). Nach Weiterdrehen der Kappe 26 um 90° stoßen die gegenüberliegend angeordneten, auf der Stirnseite des Kanülenbehälters 2 geeignet geführten Schieber 23a und 23b in der Mitte zusammen und verschießen die Durchgangsöffnung 17 und die Befestigungsöffnungen 14. Fig. 14 zeigt den Entriegelungsmechanismus 22 in der Draufsicht.

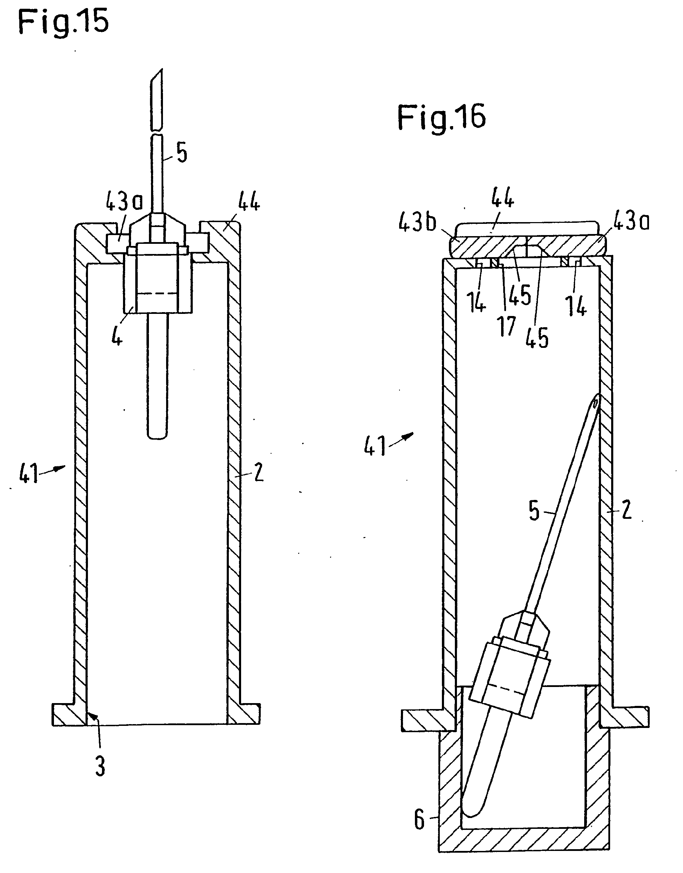

Fig. 15 zeigt eine dritte Ausführungsform eines erfindungsgemäßen Kanülenhalters 41, der dem Kanülenhalter 1 der oben beschriebenen ersten Ausführungsform im grundsätzlichen Aufbau ähnlich ist. Insbesondere der Kanülenbehälter 2 und die Kanülenhalterung 4 sind vergleichbar ausgebildet. Daher wird auf eine detaillierte Beschreibung der gleichen Teile verzichtet, die der Einfachheit halber mit denselben Bezugszeichen versehen sind.

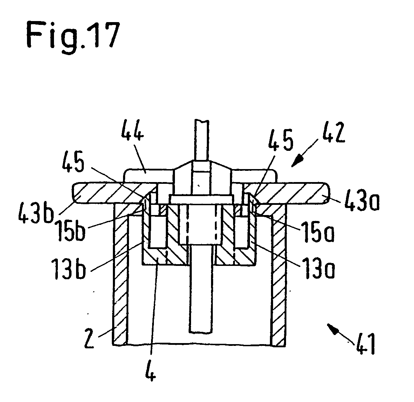

Der Kanülenhalter 41 weist einen von den Kanülenhaltern 1 und 21 verschiedenen Entriegelungsmechanismus 42 auf, der im Detail in Fig. 17 zu erkennen ist. Der Entriegelungsmechanismus 42 enthält zwei baugleiche Schieber 43a, 43b, die auf gegenüberliegenden Seiten der Kanülenhalterung 4 angeordnet sind und durch an dem Kanülenbehälter 2 angeformte Vorsprünge 44 gehalten und geführt werden. Bei eingesetzter Kanülenhalterung 4 liegen abgeschrägte Seiten 45 der Schieber 43a, 43b an den die Verankerung der Kanülenhalterung 4 bildenden Vorsprüngen 15a, 15b an.

Um die Kanülenhalterung 4 von dem Kanülenhalter 41 zu lösen, werden die beiden Schieber 43a, 43b nach innen aufeinander zu zusammen gedrückt. Dadurch werden auch die flexiblen Arme 13a, 13b der Kanülenhalterung 4 soweit zusammen gedrückt, dass die Verankerung der Vorsprünge 15a, 15b gelöst wird und die Kanülenhalterung 4 mit Kanüle 5 in den durch eine Kappe 6 verschlossenen Kanülenbehälter 2 fällt. Durch weiteres Zusammendrücken der Schieber 43a, 43b, bis diese in der Mitte des Kanülenhalters 41 zusammenstoßen, werden ferner die Befestigungsöffnungen 14 und die Durchlassöffnung 17 in der zweiten Stirnseite des Kanülenbehälters 2 zuverlässig geschlossen (vgl. Fig. 16). Im Vergleich zu dem einen Schieber 8 des Kanülenhalters 1 gemäß der ersten Ausführungsform sind die beiden Schieber 43a, 43b um 90° gedreht an dem Kanülenhalter 41 festgelegt und stehen weniger über den Rand des Kanülenbehälters 4 vor.

Durch den zuvor beschriebenen Aufbau wird ein direkt wirkender und einfacher Entriegelungsmechanismus 42 erreicht. Dies erleichtert die Handhabung in der Praxis.

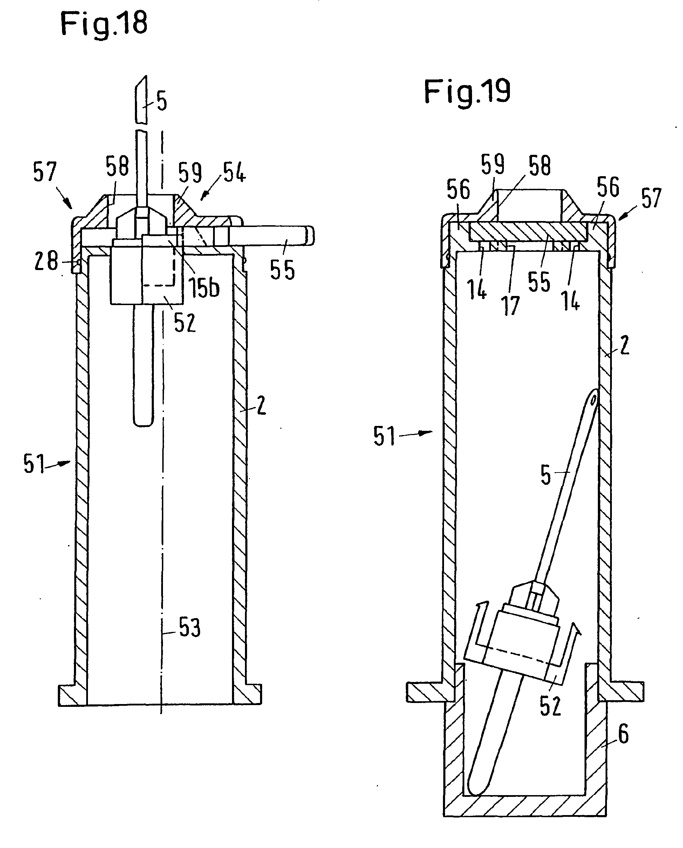

In Fig. 18 ist eine vierte Ausführungsform eines erfindungsgemäßen Kanülenhalters 51 dargestellt. Wiederum sind gleiche Teile mit zu den vorherigen Ausführungsformen identischen Bezugszeichen versehen, auf deren detaillierte Beschreibung verzichtet wird.

Der wesentliche Unterschied des Kanülenhalters 51 zu den vorher beschriebenen Ausführungsformen liegt in der Anordnung und dem Aufbau der Kanülenhalterung 52. Wie Fig. 18 zu entnehmen, sind die Kanülenhalterung 52 und die darin aufgenommene Kanüle 5 in eine radiale Richtung aus der Mittelachse 53 des Kanülenhalters 51 versetzt angeordnet. Dadurch befindet sich die Kanüle 5 näher an einem Rand des Kanülenhalters 51 und kann besser in die Vene eines Patienten eingestochen werden.

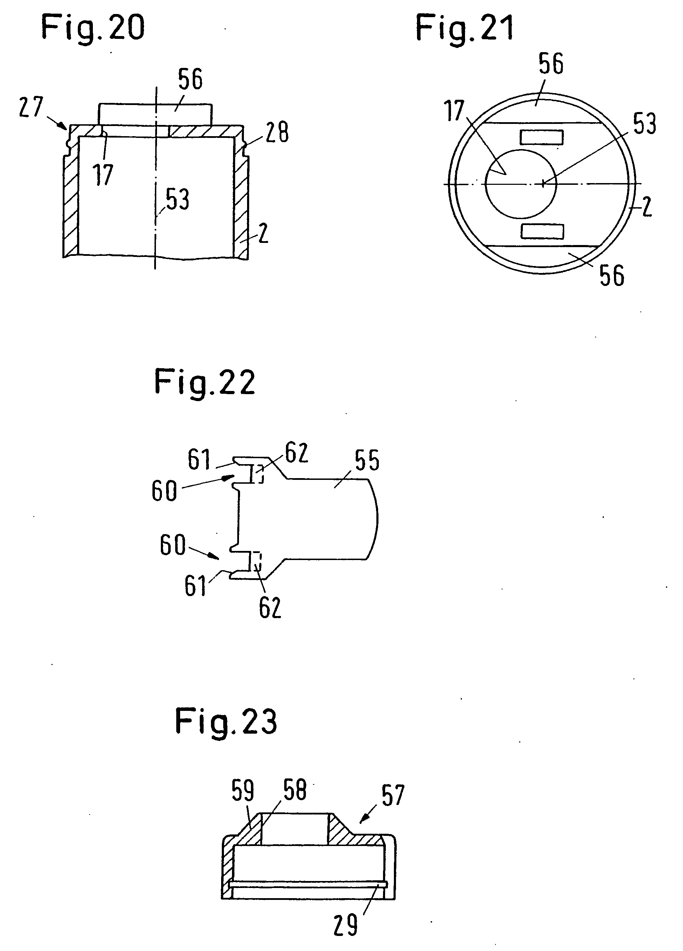

Wie Fig. 21 zu entnehmen, ist die Durchgangsöffnung 17 dazu in der Stirnseite des Kanülenbehälters 2 zu der Mittelachse 53 versetzt angeordnet. Die Befestigungsöffnungen 14 sind dagegen in ihrer Längsrichtung symmetrisch um die Mittelachse verteilt. Aufgrund dieser Anordnung ist auch die durch die Durchgangsöffnung 17 hindurchragende Kanüle 5 zum Rand des Kanülenhalters 51 hin versetzt. Der Versatz zwischen der Mittelachse 53 und dem Zentrum der Durchgangsöffnung 17 ist auch deutlich in Fig. 20 zu erkennen.

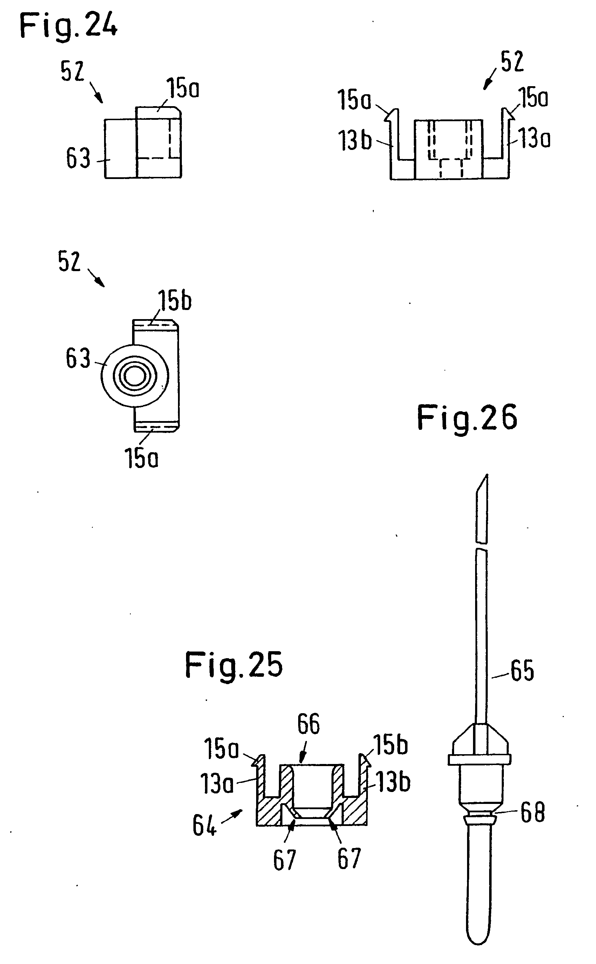

Entsprechend den vorherigen Ausführungsformen ist ein Entriegelungsmechanismus 54 mit einem Schieber 55 vorgesehen, der an den Vorsprüngen 15a, 15b der Verankerung der Kanülenhalterung 52 durch entsprechende Formgebung angreift und die Vorsprünge 15a, 15b zusammen mit den flexiblen Armen 13a, 13b zusammendrückt (vgl. Fig. 24), wenn er nach innen verschoben wird. Dadurch wird die Kanülenhalterung 52 von dem Kanülenhalter 51 gelöst und die Kanüle 5 fällt zusammen mit der Kanülenhalterung 52 in den Kanülenbehälter 2, wie in Fig. 19 dargestellt. Durch Weiterschieben des Schiebers 55 werden die Durchgangsöffnung 17 und die Befestigungsöffnungen 14 verschlossen.

Der Schieber wird seitlich durch Vorsprünge 56 an dem Kanülenbehälter 2 geführt (vgl. Fig. 20 und 21) und nach oben von einer Kappe 57 gehalten, welche an der der Durchgangsöffnung 17 entsprechenden Stelle eine Öffnung 58 mit einer Randverstärkung 59 aufweist. Die Kappe 57 wird durch Aufstecken ähnlich wie die Kappe 26 gemäß der zweiten Ausführungsform an dem Kanülenbehälter 2 festgelegt.

Der in Fig. 22 im Detail dargestellte Schieber 55 ist gabelförmig aufgebaut und weist zwei Ausnehmungen 60 auf, die an den Vorsprüngen 15a, 15b der Kanülenhalterung 52 angreifen. Dazu sind an den Ausnehmungen jeweils abgeschrägte Seiten 61, welche die Vorsprünge 15a, 15b zusammendrücken, und abgeschrägte Flächen 62 vorgesehen, welche die Vorsprünge 15a, 15b nach unten in Richtung des Kanülenbehälters 2 drücken.

Wie den Einzeldarstellung von Fig. 24 zu entnehmen, ist ein Halter 63, welcher der direkten Aufnahme der Kanüle 5 dient, versetzt zu den flexiblen Armen 13a, 13b mit den Vorsprüngen bzw. Verankerungen 15a, 15b angebracht, um im montierten Zustand entsprechend der Durchgangsöffnung 17 angeordnet zu sein.

Fig. 15 zeigt eine besondere Ausführungsform einer Kanülenhalterung 64, in die eine Kanüle 65 mittels einer Steckverbindung 66 einrastbar ist. Dazu weist die Kanülenhalterung 64 Vorsprünge 67 auf, die in eine entsprechende Nut 68 der Kanüle 65 eingreifen. Der Vorteil dieser Anordnung liegt darin, dass die Kanüle 65 in der Kanülenhalterung 64 drehbar ist. Dies ist besonders bei der versetzt angeordneten Kanülenhalterung gemäß der vierten Ausführungsform von Vorteil, um den Kanülenschliff optimal auszurichten. Die Kanülenhalterung 64 kann jedoch mit allen Kanülenhalterungen der vorbeschriebenen Ausführungsformen kompatibel ausgebildet werden.

Mit dem erfindungsgemäßen Kanülenhalter 1, 21, 41 und 51 sind eine besonders sichere, schnelle und einfache Entsorgung der Kanüle 5, 65 sowie eine einfache Handhabung gewährleistet. Da die Kanüle 5, 65 in den Halter 1, 21, 41, 51 bzw. dessen Kanülenbehälter 2 fällt, wird das Risiko einer Verletzung durch ungenaues Abwerfen ausgeschlossen und die Handhabung für das Personal somit entscheidend verbessert.

### Bezugszeichenliste

- 1: Kanülenhalter
- 2: Kanülenbehälter
- 3: Öffnung
- 4: Kanülenhalterung
- 5: Kanüle
- 6: Kappe
- 7: Entriegelungsmechanismus
- 8: Schieber
- 9a, b: Arme
- 10: Vorsprung
- 11a, b: Nut, Führung
- 12: Verankerung
- 13a, b: flexibler Arm
- 14: Befestigungsöffnung
- 15a, b: Vorsprung, Verankerung
- 16: abgeschrägte Seite
- 17: Durchgangsöffnung
- 18: Durchlass
- 19: Innengewinde

- 21: Kanülenhalter
- 22: Entriegelungsmechanismus
- 23a, b: Schieber
- 24: Ausnehmung
- 25: abgeschrägte Seite
- 26: Kappe
- 27: Absatz
- 28: Erhebung
- 29: Nut
- 30: Nippel, Führungselement
- 31: Nut

- 41: Kanülenhalter
- 42: Entriegelungsmechanismus
- 43a, b: Schieber
- 44: Vorsprung
- 45: abgeschrägte Seiten

- 51: Kanülenhalter
- 52: Kanülenhalterung
- 53: Mittelachse
- 54: Entriegelungsmechanismus
- 55: Schieber
- 56: Vorsprünge
- 57: Kappe
- 58: Öffnung
- 59: Randverstärkung
- 60: Ausnehmungen
- 61: abgeschrägte Seiten
- 62: abgeschrägte Flächen
- 63: Halter
- 64: Kanülenhalterung
- 65: Kanüle
- 66: Steckverbindung
- 67: Vorsprünge
- 68: Nut

## Patentansprüche

1. Kanülenhalter mit einem langgestreckten, an einer ersten Stirnseite eine verschließbare Öffnung (3) aufweisenden Kanülenbehälter (2), mit einer an der gegenüberliegenden zweiten Stirnseite angeordneten Kanülenhalterung (4, 52, 64) zur Aufnahme einer Kanüle (5, 65) und/oder eines Adapters zum Aufstecken einer Kanüle, welche(r) durch eine Durchgangsöffnung (17) in dem Kanülenbehälter (2) und die Kanülenhalterung (4, 52, 64) bis in den Kanülenbehälter (2) hindurchragt, und mit einem derart ausgebildeten Entriegelungsmechanismus (7, 22, 42, 54) zum Lösen der Kanüle (5, 65) und/oder des Adapters nach dem Gebrauch, dass die Kanüle (5, 65) und/oder der Adapter nach Betätigung des Entriegelungsmechanismus (7, 22, 42, 54) in den Kanülenbehälter (2) fällt, **dadurch gekennzeichnet, dass** der Entriegelungsmechanismus (7, 22, 42, 54) eine Verschlusseinrichtung (8, 23a, 23b, 43a, 43b, 55) zum Verschließen des Kanülenbehälters (2) an seiner zweiten Stirnseite aufweist, wobei die Verschlusseinrichtung (8, 23a, 23b, 43a, 43b, 55) nach Betätigung des Entriegelungsmechanismus (7, 22, 42, 54) die Durchgangsöffnung (17) für die Kanüle abdeckt.

2. Kanülenhalter nach Anspruch 1, **dadurch gekennzeichnet, dass** die Kanülenhalterung (4, 52, 64) mit einer Verankerung (12) lösbar an dem Kanülenhalter (1, 21, 41, 51) fixiert ist.

3. Kanülenhalter nach Anspruch 1 oder 2, **dadurch gekennzeichnet, dass** die Kanülenhalterung (4, 52, 64) an dem Kanülenhalter (1, 21, 41, 51) einrastbar ist.

4. Kanülenhalter nach Anspruch 2 oder 3, **dadurch gekennzeichnet, dass** der Kanülenhalter (1, 21, 41, 51) an seiner zweiten Stirnseite eine Durchgangsöffnung (17) für die Kanüle (5, 65) und/oder den Adapter und mindestens eine Befestigungsöffnung (14) für die Kanülenhalterung (4, 52, 64) aufweist.

5. Kanülenhalter nach einem der vorhergehenden Ansprüche, **dadurch gekennzeichnet, dass** die Kanülenhalterung (4, 52, 64) mindestens einen flexiblen Arm (13a, 13b) mit einem vorzugsweise keilförmigen Vorsprung (15a, 15b) zum Einrasten an dem Kanülenhalter (1, 21, 41, 51) aufweist.

6. Kanülenhalter nach einem der Ansprüche 2 bis 5, **dadurch gekennzeichnet, dass** der Entriegelungsmechanismus (7, 22, 42, 54) mindestens einen Schieber (8, 23a, 23b, 43a, 43b, 55) aufweist, welcher durch Verschieben die Verankerung (12, 15a, 15b) der Kanülenhalterung (4, 52, 64) an dem Kanülenhalter (1, 21, 41, 51) löst.

7. Kanülenhalter nach Anspruch 6, **dadurch gekennzeichnet, dass** der Schieber (8, 23a, 23b, 55) eine Führung (11a, 11b, 24, 60) aufweist, welche an der Verankerung (12, 15a, 15b) der Kanülenhalterung (4, 52, 64) angreift.

8. Kanülenhalter nach Anspruch 6 oder 7, **dadurch gekennzeichnet, dass** zwei Schieber (23a, 23b, 43a, 43b) vorgesehen sind.

9. Kanülenhalter nach einem der Ansprüche 6 bis 8, **dadurch gekennzeichnet, dass** der Schieber (8, 43a, 43b, 55) über den Rand des Kanülenbehälters (2) vorsteht.

10. Kanülenhalter nach einem der Ansprüche 6 bis 8, **dadurch gekennzeichnet, dass** der Entriegelungsmechanismus (22) eine auf dem Kanülenbehälter (2) drehbar angeordnete Kappe (26) aufweist, in welcher der mindestens eine Schieber (23a, 23b) derart aufgenommen ist, dass der Schieber (23a, 23b) durch Verdrehen der Kappe (26) bewegt wird.

11. Kanülenhalter nach Anspruch 10, **dadurch gekennzeichnet, dass** die Kappe (26) eine Führung (31) aufweist, in welche ein Führungselement (30) des Schiebers (23a, 23b) eingreift.

12. Kanülenhalter nach einem der vorhergehenden Ansprüche, **dadurch gekennzeichnet, dass** die Verschlusseinrichtung durch mindestens einen Schieber (8, 23a, 23b, 43a, 43b, 55) gebildet wird.

13. Kanülenhalter nach einem der vorhergehenden Ansprüche, **dadurch gekennzeichnet, dass** die Kanülenhalterung (52, 64) in eine radiale Richtung von der Mittelachse (53) des Kanülenhalter (51) versetzt angeordnet ist.

14. Kanülenhalter nach einem der vorhergehenden Ansprüche, **dadurch gekennzeichnet, dass** die Kanüle (65) und/oder der Adapter in der Kanülenhalterung (64) drehbar aufgenommen ist, insbesondere mittels einer Steckverbindung (66).

## Claims

1. A hollow needle holder having an elongate hollow needle container (2) comprising a closable opening (3) at a first end face, having a hollow needle fixing device (4, 52, 64) disposed on the opposite second end face for receiving a hollow needle (5, 65) and/or an adapter for attaching a hollow needle, which projects through a passage opening (17) in the hollow needle container (2) and the hollow needle fixing device (4, 52, 64) into the hollow needle container (2), and having an unlocking mechanism (7, 22, 42, 53) for releasing the hollow needle (5, 65) and/or the adapter after use, said unlocking mechanism being designed so that the hollow needle (5, 65) and/or the adapter drops into the hollow needle container (2) after the actuation of the unlocking mechanism (7, 22, 42, 54),
**characterised in that** the unlocking mechanism (7, 22, 42, 54) comprises a closure device (8, 23a, 23b, 43a, 43b, 55) for closing the hollow needle container (2) at its second end face, with the closure device (8, 23a, 23b, 43a, 43b, 55) covering the passage opening (17) for the hollow needle after the actuation of the unlocking mechanism (7, 22, 42, 54).

2. A hollow needle holder according to Claim 1,
**characterised in that** the hollow needle fixing device (4, 52, 64) is detachably fixed to the hollow needle holder (1, 21, 41, 51) by an anchoring device (12).

3. A hollow needle holder according to Claim 1 or 2,
**characterised in that** the hollow needle fixing device (4, 52, 64) can be locked on the hollow needle holder (1, 21, 41, 51).

4. A hollow needle holder according to Claim 2 or 3,
**characterised in that** the hollow needle holder (1, 21, 41, 51) comprises at its second end face a passage opening (17) for the hollow needle (5, 65) and/or the adapter and at least one attachment opening (14) for the hollow needle fixing device (4, 52, 64).

5. A hollow needle holder according to one of the preceding Claims,
**characterised in that** the hollow needle fixing device (4, 52, 64) comprises at least one flexible arm (13a, 13b) having a preferably wedge-shaped projection (15a, 15b) for engagement on the hollow needle holder (1, 21, 41, 51).

6. A hollow needle holder according to one of Claims 2 to 5,
**characterised in that** the unlocking mechanism (7, 22, 42, 54) comprises at least one slide (8, 23a, 23b, 43a, 43b, 55), which releases the anchoring device (12, 15a, 15b) of the hollow needle fixing device (4, 52, 64) on the hollow needle holder (1, 21, 41, 51) by displacement.

7. A hollow needle holder according to Claim 6,
**characterised in that** the slide (8, 23a, 23b, 55) comprises a guide (11a, 11b, 24, 60) that acts on the anchoring device (12, 15a, 15b) of the hollow needle fixing device (4, 52, 64).

8. A hollow needle holder according to Claim 6 or 7,
**characterised in that** two slides (23a, 23b, 43a, 43b) are provided.

9. A hollow needle holder according to one of Claims 6 to 8,
**characterised in that** the slide (8, 43a, 43b, 55) projects beyond the edge of the hollow needle container (2).

10. A hollow needle holder according to one of Claims 6 to 8,
**characterised in that** the unlocking mechanism (22) comprises a cap arranged to rotate on the hollow needle container (2), in which cap the at least one slide (23a, 23b) is housed in such a manner that the slide (23a, 23b) is moved by twisting the cap (26).

11. A hollow needle holder according to Claim 10,
**characterised in that** the cap (26) comprises a guide (31) into which a guide element (30) of the slide (23a, 23b) engages.

12. A hollow needle holder according to one of the preceding Claims,
**characterised in that** the closure device is formed by at least one slide (8, 23a, 23b, 43a, 43b, 55).

13. A hollow needle holder according to one of the preceding Claims,
**characterised in that** the hollow needle fixing device (52, 64) is arranged offset from the centre axis (53) of the hollow needle holder (51) in a radial direction.

14. A hollow needle holder according to one of the preceding Claims,
**characterised in that** the hollow needle (65) and/or the adapter is mounted in the hollow needle fixing device (64) to rotate, in particular by means of a plug-in connector (66).

## Revendications

1. Support de canule avec un réceptacle à canule (2) longiforme présentant à une première face frontale une ouverture (3) pouvant être fermée, avec un porte-canule (4, 52, 64) disposé sur la deuxième face frontale opposée à la première pour recevoir une canule (5, 65) et/ou un adaptateur pour embrocher une canule, laquelle/lequel dépasse jusque dans le réceptacle à canule (2) en passant par une ouverture de passage (17) située dans le réceptacle à canule (2) et par le porte-canule (4, 52, 64), et avec un mécanisme de déverrouillage (7, 22, 42, 54) pour libérer après usage la canule (5, 65) et/ou l'adaptateur et conçu de telle sorte que la canule (5, 65) et/ou l'adaptateur tombe dans le réceptacle à canule (2) après actionnement du mécanisme de déverrouillage (7, 22, 42, 54), **caractérisé en ce que** le mécanisme de déverrouillage (7, 22, 42, 54) est muni d'un dispositif de fermeture (8, 23a, 23b, 43a, 43b, 55) pour fermer le réceptacle à canule (2) au niveau de sa deuxième face frontale, le dispositif de fermeture (8, 23a, 23b, 43a, 43b, 55) couvrant l'ouverture de passage (17) pour la canule après l'actionnement du mécanisme de déverrouillage (7, 22, 42, 54).

2. Support de canule selon la revendication 1, **caractérisé en ce que** le porte-canule (4, 52, 64) est fixé de façon réversible sur le support de canule (1, 21, 41, 51) avec un ancrage (12).

3. Support de canule selon la revendication 1 ou 2, **caractérisé en ce que** le porte-canule (4, 52, 64) peut être encliqueté sur le support de canule (1, 21, 41, 51).

4. Support de canule selon la revendication 2 ou 3, **caractérisé en ce que** le support de canule (1, 21, 41, 51) est muni sur sa deuxième face frontale d'une ouverture de passage (17) pour la canule (5, 65) et/ou l'adaptateur et d'au moins d'une ouverture de fixation (14) pour le porte-canule (4, 52, 64).

5. Support de canule selon l'une des revendications précédentes, **caractérisé en ce que** le porte-canule (4, 52, 64) est muni d'au moins un bras flexible (13a, 13b) avec une saillie (15a, 15b) de préférence en forme de coin pour l'encliquetage sur le support de canule (1, 21, 41, 51).

6. Support de canule selon l'une des revendications 2 à 5, **caractérisé en ce que** le mécanisme de déverrouillage (7, 22, 42, 54) est muni au moins d'un poussoir (8, 23a, 23b, 43a, 43b, 55) qui par déplacement libère l'ancrage (12, 15a, 15b) du porte-canule (4, 52, 64) sur le support de canule (1, 21, 41, 51).

7. Support de canule selon la revendication 6, **caractérisé en ce que** le poussoir (8, 23a, 23b, 55) est muni d'un guide (11a, 11b, 24, 60) qui se met en prise avec l'ancrage (12, 15a, 15b) du porte-canule (4, 52, 64).

8. Support de canule selon la revendication 6 ou 7, **caractérisé en ce que** deux poussoirs (23a, 23b, 43a, 43b) sont prévus.

9. Support de canule selon l'une des revendications 6 à 8, **caractérisé en ce que** le poussoir (8, 43a, 43b, 55) dépasse au-dessus du bord du réceptacle à canule (2).

10. Support de canule selon l'une des revendications 6 à 8, **caractérisé en ce que** le mécanisme de déverrouillage (22) est muni d'une calotte (26) disposée pivotante sur le réceptacle à canule (2) dans laquelle le au moins un poussoir (23a, 23b) est reçu de telle sorte que le poussoir (23a, 23b) est déplacé en tournant la calotte (26).

11. Support de canule selon la revendication 10, **caractérisé en ce que** la calotte (26) est munie d'un guide (31) dans lequel s'engage un élément de guidage (30) du poussoir (23a, 23b).

12. Support de canule selon l'une des revendications précédentes, **caractérisé en ce que** le dispositif de fermeture est formé par au moins un poussoir (8, 23a, 23b, 43a, 43b, 55).

13. Support de canule selon l'une des revendications précédentes, **caractérisé en ce que** le porte-canule (52, 64) est disposé de façon décalée dans une direction radiale par rapport à l'axe médian (53) du support de canule (51).

14. Support de canule selon l'une des revendications précédentes, **caractérisé en ce que** la canule (65) et/ou l'adaptateur est reçu pivotant dans le porte-canule (64), notamment au moyen d'une liaison enfichable (66).
